# EUROPEAN PATENT SPECIFICATION

(11) **EP 1 481 668 B1**
(45) Date of publication and mention of the grant of the patent: **02.01.2008**
(21) Application number: 04076366.6
(22) Date of filing: 06.05.2004
(51) Int. Cl.: A61K 31/00, A61K 31/197, A61P 25/08

(54) **Use of anti-glaucoma drugs to treat visual field defects associated with the use of a GABAergic agent**
Verwendung von Antiglaukomamitteln zur Behandlung von Gesichtsfeldausfällen hervorgerufen durch GABA-ergen Wirkstoffen
Utilisation d'agents antiglaucomateux pour le traitement de défauts du champ visuel liés à l'utilisation d'un agent GABA-ergique

(30) Priority: 27.05.2003 US 446285
(43) Date of publication of application: 01.12.2004
(73) Proprietor: Brookhaven Science Associates, Upton, New York 11973 (US)
(72) Inventor: Ashby, Charles R. Jr., Sound Beach, NY 11789 (US)
(74) Representative: Winckels, Johannes Hubertus F.

(56) References cited:
- HOSKING, S. L. ET AL.: "Epilepsy patients treated with vigabatrin exhibit reduced ocular blood flow" BRITISH JOURNAL OF OPHTHALMOLOGY, vol. 87, no. 1, 1 January 2003 (2003-01-01), pages 96-100, XP002290634 UK
- LAWDEN, M., C. ET AL.: "Visual field defects associated with vigabatrin therapy" JOOURNAL OF NEUROLOGY NEUSURGERY AND PSYCHIATRY, vol. 67, no. 6, 1999, pages 716-722, XP002290635 UK
- SPONSEL W E ET AL: "ASSOCIATION OF RETINAL CAPILLARY PERFUSION WITH VISUAL STATUS DURING CHRONIC GLAUCOMA THERAPY" OPHTHALMOLOGY, J. B. LIPPINCOTT CO., PHILADELPHIA, PA, US, vol. 104, no. 6, 1997, pages 1026-1032, XP009033279 ISSN: 0161-6420
- SATO T ET AL: "SHORT-TERM EFFECT OF BETA-ADRENORECEPTOR BLOCKING AGENTS ON OCULAR BLOOD FLOW" CURRENT EYE RESEARCH, IRL PRESS, OXFORD, GB, vol. 23, no. 4, 2002, pages 298-306, XP009033397 ISSN: 0271-3683
- WOLF S ET AL: "EINFLUSS VON BETA-BLOCKERN AUF DIE RETINALE HAEMODYNAMIK DOPPELBLINDSTUDIE BEI GESUNDEN INFLUENCE OF BETA-ADRENERGIC BLOCKERS ON RETINAL HEMODYNAMICS: A DOUBLE-BLIND STUDY WITH HEALTHY VOLUNTEERS" KLINISCHE MONATSBLAETTER FUER AUGENHEILKUNDE, FERDINAND ENKE VERLAG, STUTTGART, DE, vol. 195, no. 4, 1989, pages 229-231, XP009033398 ISSN: 0023-2165
- HARRIS A ET AL: "THE IMPACT OF GLAUCOMA MEDICATION ON PARAMETERS OF OCULAR PERFUSION" CURRENT OPINION IN OPHTHALMOLOGY, PHILADELPHIA, PA, US, vol. 12, no. 2, 2001, pages 131-137, XP009033402 ISSN: 1040-8738

## Description

### BACKGROUND OF THE INVENTION

The invention relates to the field of treating visual field defects associated with the use of GABAergic drugs, in mammals.

GABAergic drugs are known to increase GABA levels in organs, such as the brain and eye. These drugs are well known to be effective in the treatment of conditions such as seizure disorders including, for example, infantile spasms and epilepsy.

Recently, it has been reported that some GABAergic drugs such as, for example, gamma-vinyl GABA (GVG), are also effective for treating and preventing drug addiction. See U.S. Patent Nos. 6,057,368, 6,395,783, and 6,541,520 to Dewey et al., and pending U.S. Patent Application Nos. 09/189,166; 09/362,592; and 09/853,548.

Typically, GVG functions by irreversibly inhibiting the GABA-degrading enzyme GABA transaminase (GABA-T). The inhibition of GABA-T generally results in an increase in GABA levels. Furthermore, it has been shown that GVG-induced increases in GABA levels cause a decrease in glutamate decarboxylase (GAD), a GABA synthesizing enzyme.

It has been realized, however, that visual disturbances have been associated with the use of GABAergic drugs. In particular, there have been reports that approximately 25-50% of patients treated with GVG develop visual field defects (VFDs). Although the mechanism responsible for GVG-induced VFDs is unknown, it is believed that the VFDs induced by GVG may be related to elevated levels of GABA within the retina. See, e.g. Comaish, et al., "The effects of vigabatrin on electrophysiology and visual field in epileptics: a controlled study with a discussion of possible mechanisms." Doc. Opthalmol. 104:195-212 (2002).

One proposed method for treating or preventing VFDs associated with the use of GABAergic drugs, such as GVG, involves the administration of vitamin B6. See U.S. Application No. 10/389,578, filed March 17, 2003. It is believed that vitamin B6 mitigates the visual field defects by promoting GABA-T, which in turn degrades any excess GABA.

Hosking et al., Br. J. Ophthalmol. 2003; 87: 96-100, describes that epilepsy patients treated with vigabatrin exhibit reduced ocular blood flow.

Lawden et al., J. Neurol. Neurosurg. Psychiatry 1999; 67: 716-722 describes the occurrence of visual field defects associated with vigabatrin therapy.

Sponsel et al., Ophthalmology 1997; 104: 1026-1032 describes an association of retinal capillary perfusion with visual status during chronic glaucoma therapy.

Sato et al., Current Eye Research 2001; 23: 298-306 describes a short-term effect of β-adrenoreceptor blocking agents on ocular blood flow.

Wolf et al., Klin. Mbl. Augenheilk. 1989; 195: 229-231 describes an influence of beta-blocker on the retinal haemodynamics.

Harris et al., Current Opinion in Ophthalmology 2001; 12: 131-137 describes the impact of glaucoma medication on parameters of ocular perfusion.

There exists a need for alternative approaches to preventing and/or treating VFDs associated with the use of GABAegic drugs.

It is thus an object of the present invention to provide compositions and methods for treating and/or preventing VFDs associated with the use of GABAegic drugs, such as GVG.

### SUMMARY OF THE INVENTION

These and other objectives have been met by the present invention. The invention provides the use of a drug that improves retinal perfusion for preparing a pharmaceutical composition for ameliorating visual field defects associated with concurrent or subsequent administration of a GABAergic drug to a mammal in need thereof. The composition may be administered to the mammal in an effective amount of the drug that improves retinal perfusion.

In another embodiment, the invention provides a composition containing a GABAergic drug, and an effective amount of a drug that improves retinal perfusion.

### DETAILED DESCRIPTION OF THE INVENTION

The present invention is for novel compositions and uses related to treating visual field defects associated with the use of GABAergic drugs, in a mammal in need thereof.

By "treating" is meant administering to a mammal a therapeutically effective amount of a drug that improves retinal perfusion so that the visual field defects are at least partially and/or substantially completely alleviated in the mammal.

In addition, treating means administering a therapeutically effective amount of a drug that improves retinal perfusion so that the visual field defects are at least partially and/or substantially completely prevented from occurring in the mammal.

A "GABAergic drug" is any compound that potentiates the GABAergic system or increases gamma amino butyric acid (GABA) levels in the central nervous system (CNS). According to the invention, the CNS includes the spinal cord, brain and midbrain regions. GABA is a widespread inhibitory neurotransmitter in the CNS. GABA is made in the brain from the amino acid glutamate with the aid of vitamin B6.

GABAergic drugs include compounds that enhance the production or release of GABA in the CNS and/or increase GABAergic transmission. In addition, a GABAergic drug is any compound that directly or indirectly augments or facilitates GABAergic neurotransmission in the CNS and/or the retina and/or the optic nerve.

These drugs include, but are not limited to, gabapentin, valproic acid, progabide, gamma-hydroxybutyric acid, fengabine, cetylGABA, topiramate, tiagabine, acamprosate (homo-calcium-acetyltaurine) or pharmaceutically acceptable salts thereof, or enantiomers or racemic mixtures thereof.

A GABAergic drug is meant to include the pharmaceutically acceptable salts of the drug. As used herein, pharmaceutically acceptable salts include those salt-forming acids and bases which do not substantially increase the toxicity of the compound. Some examples of suitable salts include salts of mineral acids such as hydrochloric, hydriodic, hydrobromic, phosphoric, metaphosphoric, nitric and sulfuric acids, as well as salts of organic acids such as tartaric, acetic, citric, malic, benzoic, glycollic, gluconic, gulonic, succinic, arylsulfonic, e.g. p-toluenesulfonic acids, and the like.

The present invention embraces compositions which include prodrugs of GABA or drugs which contain GABA as a moiety in its chemical structure. These prodrugs become pharmacologically active when metabolically, enzymatically or non-enzymatically biotransformed or cleaved into GABA in the CNS. An example of a prodrug of GABA is progabide, which upon crossing the blood brain barrier, increases endogenous CNS GABA levels.

A preferred GABAergic drug is gamma vinyl GABA (GVG). GVG is represented by the compound 4-amino-hex-5-enoic acid, and is sold under the product names Vigabatrin® and Sabril® by Hoechst-Marion Roussel. GVG is an irreversible inhibitor of the pyridoxal-phoshate dependent enzyme GABA transaminase (GABA-T) and is responsible for metabolizing GABA to succinic semialdehyde. GVG is also known to inhibit other transaminases, such as, for example, alanine aminotransferase and ornithine aminotransferase.

Not being bound by theory, it is believed that GVG's action results in an accumulation of GABA within the nerve terminal, and ultimately, an increase in synaptic GABA levels. GVG has also been reported to produce changes in the content/level of other amino acids in the brain such as, for example, a decrease in glutamate, aspartate and glutamine, and an increase in cerebrospinal fluid levels of homocarnosine.

GVG does not bind to any receptor or reuptake complex, but typically increases endogenous intracellular GABA levels by selectively and irreversibly inhibiting GABA-transaminase (GABA-T), the enzyme that normally catabolizes GABA.

As used herein, GVG includes the racemic compound or mixture which contains equal amounts of S(+)-gamma-vinyl GABA, and R(-)-gamma vinyl GABA. This racemic compound of GVG is available as Vigabatrin^{®} from Hoechst Marion Roussel and can be obtained from Marion Merell Dow of Cincinnati, Ohio.

GVG contains asymmetric carbon atoms and thus is capable of existing as enantiomers. The present invention embraces any enantiomeric form of GVG including the racemates or racemic mixture of GVG. In some cases there may be advantages, i.e. greater efficacy, to using a particular enantiomer when compared to the other enantiomer or the racemate or racemic mixture in the methods of the instant invention and such advantages can be readily determined by those skilled in the art.

For example, the enantiomer S(+)-gamma-vinyl GABA is more effective at increasing endogenous intracellular GABA levels than the enantiomer R(-)-gamma-vinyl GABA.

Different enantiomers may be synthesized from chiral starting materials, or the racemates may be resolved by conventional procedures which are well known in the art of chemistry such as chiral chromatography, fractional crystallization of diastereomeric salts, and the like.

According to the invention, "a mammal in need thereof" is any mammal that has previously taken, is currently taking, or will be taking, a GABAergic drug, for any condition.

Such conditions include, for example, seizure disorders and drug addiction. Exemplary seizure disorders include, but are not limited to epilepsy and West syndrome (i.e. infantile spasms).

"Drug addiction" is defined as addiction to one or more drugs of abuse. Drugs of abuse include, but are not limited to, stimulants such as cocaine, amphetamine, pipradol, methylphenidate, nicotine and caffeine, narcotics and pain medications such as morphine and methadone, and central nervous system depressants such as barbiturates, chlordiazepoxide and ethanol.

According to the present invention, addiction to a combination of one or more drugs of abuse is also a condition that is treated by administering a GABAergic drug.

A "mammal" suitable for the methods of the present invention is any mammal, including a human, domestic animal (e.g. cat or dog), laboratory animal (e.g. rat or monkey) or farm animal (e.g. cow, horse or pig). Most preferably the mammal is a human. All humans are contemplated, including infants, children and adults.

In accordance with the invention, "visual field defects" associated with the use of a GABAergic drug include any disturbance of the visual field. For example, visual field defects include loss of visual acuity, loss of peripheral vision, visual field constrictions, reduction in retinal cone function, bilateral concentric defect and optic neuropathy. Typically, visual field defects are associated with the retina.

Not being bound by theory, it is believed that GABAergic drugs may produce visual field defects by directly or indirectly altering blood flow to the retina. For example, the GABAergic drug may increase intraocular pressure in the eye by, for instance, decreasing or preventing fluid draining from the eye. An increase in intraocular pressure can, for example, decrease blood flow to the retina. A decrease in retinal blood flow can lead to a degeneration of optic nerve function, thus eliciting visual field defects.

The visual field defects associated with use of GABAergic drugs such as, for example, GVG, may also be idiopathic in nature, i.e. no known cause can be identified.

According to the invention, improving retinal perfusion means increasing blood flow to the retina, which was altered (e.g., decreased) directly or indirectly by the use of GABAergic drugs. The improvement in retinal perfusion can prevent, diminish or eliminate visual field defects associated with the use of a GABAergic drug.

The drug can be any drug that improves retinal perfusion. Drugs that improve retinal perfusion include any drug that increases blood flow to the retina. Examples of drugs that increase blood flow to the retina include, but are not limited to pentoxifylline, puerarin derivatives, nitropyrazole derivatives, inhibitors of nitric oxide synthase, and anti-glaucoma drugs.

Anti-glaucoma drugs include, for example, beta-blockers, prostaglandin analogs, adrenergic agonists, cholinergic agonists, carbonic anhydrase inhibitors, and alpha-adrenergic agonists.

Beta-blockers and adrenergic agonists essentially block beta adrenergic substances such as adrenaline (e.g. epinephrine). Some examples of beta-blockers include betaxolol, cartelol, levobunolol, metipranolol, timolol, levobetaxolol, and propranolol. Examples of adrenergic agonists include epinephrine and dipivefrin

Prostaglandin analogs lower intraocular pressure by increasing the outflow of the fluid that the eye continually makes called the aqueous humor. Examples of prostaglandin analogs include latanoprost, unoprostone, bimatoprost, and travopost.

Cholinergic drugs act by mimicking the effects of the chemical acetylcholine, which controls the production of fluid in the eye. Examples of cholinergic agonists include pilocarpine, carbochol, demacrium, echothiophate iodide, and physostigmine.

Carbonic anyhdrase inhibitors work by inhibiting the ciliary process in the eye which decreases the aqueous humor secretion, presumably by slowing down the formation of bicarbonate ions with the subsequent reduction in sodium and fluid transport. Examples of carbonic anhydrase inhibitors include dorzolamide, brinzolamide, acetazolamide, and methazolamide.

Alpha-adrenergic agonists work by lowering intraocular pressure by reducing the production of aqueous flow. Examples of alpha-adrenergic agonists include apraclonidine, brimonidine.

The drug that improves retinal perfusion can be used alone or in combination with another drug that also improves retinal perfusion. For example, a beta-blocker can be used in combination with a prostaglandin analog. Alternatively, two different beta-blocker drugs can be used in combination.

The drug that improves retinal perfusion is administered in any form including tablet, caplet, and liquid formulations. The drug can be administered systemically or optically (e.g., eye drops). Optical administration is preferred.

Systemic administration can be achieved by any means known to the skilled practitioner, including enteral and parenteral administration. The drug can also be suitably administered nasally, transdermally or by nebulizer. Sustained release formulations are also contemplated by the invention, and are discussed below.

According to the invention, an "effective amount" of a drug that improves retinal perfusion is administered to a mammal. An effective amount of the drug is an amount that is effective to treat and/or prevent visual defects associated with the use of a GABAergic drug.

An effective amount of the drug easily be determined by the skilled practitioner. Exemplary dosages of optical solutions of the following drugs are provided.

For example, betaxolol (sold as Betoptic®) is administered in a concentration of approximately 0.25% to 0.5%, twice daily. Carteolol (sold as Ocuporess®) is administered in a concentration of approximately 1.0%, twice daily. Levobubulol (sold as Betagan®) is administered in a concentration of approximately 0.25% to 0.5% once or twice daily. Timolol (sold as Timoptic®/Betimol®) is administered in a concentration of approximately 0.25% to 0.5% twice daily. Levobetaxolol (sold as Betaxon®) is administered in a concentration of approximately 0.50% twice daily.

Epinephrine (sold as Epifren®) is administered in a concentration of approximately 0.1% to 0.2% once or twice daily. Dipivefrin (sold as Propine®) is administered in a concentration of approximately 0.10% every 12 hours.

Acetazolamide (sold as Diamox®) is administered in a concentration of approximately 250mg four times a day or 250mg, sustained release, twice a day. Methazolamide (soled as Neptazane®) is administered in a concentration of approximately 25 to 100 mg three times a day.

Pilocarpine (sold as Isopto Carpine®, Pilocar®, or Pilostat®) is administered in a concentration of approximately 0.25% to 10% two to four times daily. Carbachol (sold as Isopto Carbachol® or Carboptic®) is administered in a concentration of approximately 0.75% to 3% three times daily. Demacarium (sold as Humursol®) is administered in a concentration of approximately 0.125% to 0.25% twice daily.

Echothiophate Iodide (sold as Phosholine Iodide©) is administered in a concentration of approximately 0.03% to 0.25% twice daily. Physostigmine (sold as Isopto Eserine®) is administered in a concentration of approximately 0.25% to 0.5% three to four times daily.

Dorzolamide (sold as Trusopt®) is administered in a concentration of approximately 2% three times daily. Brinzolamide (sold as Azopt®) is administered in a concentration of approximately 1% three times daily.

Latanoprost (sold as Xalatan®) is administered in a concentration of approximately 0.01% once daily. Unoprostone (sold as Resula®) is administered in a concentration of approximately 0.15% twice daily. Bimatoprost (sold as Lumigan®) is administered in a concentration of approximately 0.03% once daily. Travaprost (sold as Travatan®) is administered in a concentration of approximately 0.004% once daily.

Apraclonidine (sold as Iopidine®) is administered in a concentration of approximately 0.5% to 0.20% three times daily. Brimonidine (sold as Alphagan®) is administered in a concentration of approximately 0.20% three times daily.

Dorzolamide, also known as timilol (both are sold as Cosopt®) are administered in concentrations of approximately 2% and 0.5%, respectively, two times daily. Epinephrine (sold as EP®), also known as pilocarpine (sold as E-Pilo®), are administered in concentrations of approximately 1% once daily, and 1-6% four times daily, respectively.

According to the invention, the drug can be administered prior to, simultaneously with, or after, administration of a GABAergic drug. For example, prior to beginning treatment with a GABAergic drug, a mammal can begin to take a drug that improves retinal perfusion.

Prior administration of the drug can begin at any time before commencement of treatment with a GABAergic drug. Preferably, the drug is administered from about one month, more preferably about two weeks, and even more preferably about one day, before commencement of treatment with a GABAergic drug. Simultaneous administration is accomplished by beginning treatment of the GABAergic drug at the same time treatment with the drug is commenced.

Commencement of the administration of the drug can occur at any time after treatment with a GABAergic drug. Preferably, commencement the drug is administered within about one year after treatment with a GABAergic drug, more preferably within about six months, even more preferably within about one month, and most preferably within about one day after treatment with a GABAergic drug.

The mammal may or may not continue treatment with the drug after treatment with the GABAergic drug ends. Such continued treatment can last for anytime up to about one year after treatment with the GABAergic drug ceases. Alternatively, the mammal can cease taking the drug at the same time the GABAergic drug is ceased.

In another embodiment, the invention provides a novel composition containing a GABAergic drug and a drug that improves retinal reperfusion, in a suitable pharmaceutical carrier (vehicle) or excipient as understood by practitioners in the art.

Exemplary drugs that improve retinal reperfusion are discussed above. Any of these aforementioned drugs is suitable for the composition.

Examples of pharmaceutically acceptable carriers and excipients include starch, milk, sugar, certain types of clay, gelatin, stearic acid or salts thereof, magnesium or calcium stearate, talc, vegetable fats or oils, gums and glycols.

The compositions of the invention may be administered by methods known in the art, typically, systemically. Systemic administration can be enteral or parenteral. Enteral administration is a preferred route of delivery of the compositions. Liquid or solid (e.g., tablets, gelatin capsules) formulations can be employed.

Administration can also be accomplished by a nebulizer or liquid mist. Optical administration is preferred (e.g. eye drops). Parenteral administration of the compositions of the invention (e.g., intravenous, intramuscular, subcutaneous injection) is also contemplated. Formulations using conventional diluents, carriers, etc. such as are known in the art can be employed to deliver the compound.

The compositions may be in a sustained release formulation, as is known in the art. Sustained release administration, as discussed above, allows one to achieve a certain level of the drug over a particular period of time.

Preferably, the composition contains a GABAergic drug in an amount which is effective for the purpose intended, such as treatment of seizure disorder or drug addiction, but has little or no adverse effects. For example, the amount of GVG in the composition is from about 15mg/kg to about 2g/kg or from about 15mg/kg to about 600mg/kg.

A composition containing gabapentin will include gabapentin in an amount from about 500mg to about 2g/day. Gabapentin is available as Neurontin^{®} from Parke-Davis in the United States.

A composition containing valproic acid will include valproic acid in an amount from about 5mg/kg to about 100 mg/kg/day. Valproic acid is available as Depakene^{®} from Abbott in the United States.

A composition containing topiramate acid will include topiramate in an amount from about 50mg to about 1g/day. is available as Topamax^{®} from McNeil in the United States.

A composition containing progabide will include progabide in an amount from about 250mg to about 2g/day. Progabide is available as Gabrene^{®} from Synthelabo, France. The chemical formula of progabide is C₁₇ H₁₆ N₂ O₂.

A composition containing fengabine will include fengabine in an amount from about 250mg to about 4g/day. Fengabine is available as SL 79229 from Synthelabo, France. The chemical formula of fengabine is C₁₇ H₁₇ C₁₂ NO.

A composition containing gamma-hydroxybutyric acid will include gamma-hydroxybutyric acid in an amount from about 5mg/kg to about 100mg/kg/day. Gamma-hydroxybutyric acid is available from Sigma Chemical. The chemical formula of gamma-hydroxybutyric acid is C₄ H₇ O₃ Na.

The amount of a drug that improves retinal perfusion in the composition can vary and may depend upon the amount of GABAergic drug administered and the mammal being treated. Examples of suitable amounts of drugs that improve retinal reperfusion have been disclosed above, and can be further determined by those skilled in the art during pre-clinical and clinical trials.

## Claims

1. Use of a drug that improves retinal perfusion for preparing a pharmaceutical composition for ameliorating visual field defects associated with concurrent or subsequent administration of a GABAergic drug to a mammal in need thereof.

2. Use according to claim 1, wherein the GABAergic drug is gamma vinyl GABA.

3. Use according to claim 1, wherein the drug that improves retinal perfusion is a beta blocker.

4. Use according to claim 1, wherein the drug that improves retinal perfusion is a prostaglandin analog.

5. Use according to claim 1, wherein the drug that improves retinal perfusion is an adrenergic agonist.

6. Use according to claim 1, wherein the drug that improves retinal perfusion is a cholinergic agonist.

7. Use according to claim 1, wherein the drug that improves retinal perfusion is a carbonic anhydrase inhibitor.

8. Use according to claim 1, wherein the drug that improves retinal perfusion is an alpha-adrenergic agonist.

9. Use according to claim 1, wherein the drug that improves retinal perfusion is selected from the group consisting of pentoxifylline, puerarin derivatives, nitropyrazole derivatives, inhibitors of nitric oxide synthase and combinations thereof.

10. Use according to claim 1, wherein the mammal is a human.

11. A composition comprising a GABAergic drug and an effective amount of a drug that improves retinal perfusion.

12. A composition according to claim 11, wherein the drug that improves retinal perfusion is a beta blocker.

13. A composition according to claim 11, wherein the drug that improves retinal perfusion is a prostaglandin analog.

14. A composition according to claim 11, wherein the drug that improves retinal perfusion is an adrenergic agonist.

15. A composition according to claim 11, wherein the drug that improves retinal perfusion is a cholinergic agonist.

16. A composition according to claim 11, wherein the drug that improves retinal perfusion is a carbonic anhydrase inhibitor.

17. A composition according to claim 11, wherein the drug that improves retinal perfusion is an alpha-adrenergic agonist.

18. A composition according to claim 11, wherein the drug that improves retinal perfusion is selected from the group consisting of pentoxifylline, puerarin derivatives, nitropyrazole derivatives, inhibitors of nitric oxide synthase and combinations thereof.

19. A composition according to claim 11, wherein the GABAergic drug is gamma vinyl GABA.

## Patentansprüche

1. Verwendung eines Arzneimittels, welches die retinale Durchblutung verbessert, für die Herstellung einer pharmazeutischen Zusammensetzung zur Verbesserung von Gesichtsfeldausfällen, die in Zusammenhang stehen mit der gleichzeitigen oder nachfolgenden Verabreichung eines GABAergen Arzneimittels an einen Säuger, der dieses benötigt.

2. Verwendung nach Anspruch 1, worin das GABAerge Arzneimittel Gamma-Vinyl-GABA ist.

3. Verwendung nach Anspruch 1, worin das Arzneimittel, welches die retinale Durchblutung verbessert, ein Betablocker ist.

4. Verwendung nach Anspruch 1, worin das Arzneimittel, welches die retinale Durchblutung verbessert, ein Prostaglandin-Analogon ist.

5. Verwendung nach Anspruch 1, worin das Arzneimittel, welches die retinale Durchblutung verbessert, ein adrenerger Agonist ist.

6. Verwendung nach Anspruch 1, worin das Arzneimittel, welches die retinale Durchblutung verbessert, ein cholinerger Agonist ist.

7. Verwendung nach Anspruch 1, worin das Arzneimittel, welches die retinale Durchblutung verbessert, ein Carboanhydrase-Inhibitor ist.

8. Verwendung nach Anspruch 1, worin das Arzneimittel, welches die retinale Durchblutung verbessert, ein alpha-adrenerger Agonist ist.

9. Verwendung nach Anspruch 1, worin das Arzneimittel, welches die retinale Durchblutung verbessert, ausgewählt wird aus der Gruppe bestehend aus Pentoxifyllin, Puerarin-Derivaten, Nitropyrazol-Derivaten, Inhibitoren der Stickstoffoxid-Synthase und Kombinationen davon.

10. Verwendung nach Anspruch 1, worin der Säuger ein Mensch ist.

11. Zusammensetzung, umfassend ein GABAerges Arzneimittel und eine wirksame Menge eines Arzneimittels, welches die retinale Durchblutung verbessert.

12. Zusammensetzung nach Anspruch 11, worin das Arzneimittel, welches die retinale Durchblutung verbessert, ein Betablocker ist.

13. Zusammensetzung nach Anspruch 11, worin das Arzneimittel, welches die retinale Durchblutung verbessert, ein Prostaglandin-Analogon ist.

14. Zusammensetzung nach Anspruch 11, worin das Arzneimittel, welches die retinale Durchblutung verbessert, ein adrenerger Agonist ist.

15. Zusammensetzung nach Anspruch 11, worin das Arzneimittel, welches die retinale Durchblutung verbessert, ein cholinerger Agonist ist.

16. Zusammensetzung nach Anspruch 11, worin das Arzneimittel, welches die retinale Durchblutung verbessert, ein Carboanhydrase-Inhibitor ist.

17. Zusammensetzung nach Anspruch 11, worin das Arzneimittel, welches die retinale Durchblutung verbessert, ein alpha-adrenerger Agonist ist.

18. Zusammensetzung nach Anspruch 11, worin das Arzneimittel, welches die retinale Durchblutung verbessert, ausgewählt wird aus der Gruppe bestehend aus Pentoxifyllin, Puerarin-Derivaten, Nitropyrazol-Derivaten, Inhibitoren der Stickstoffoxid-Synthase und Kombinationen davon.

19. Zusammensetzung nach Anspruch 11, worin das GABAerge Arzneimittel Gamma-Vinyl-GABA ist.

## Revendications

1. Utilisation d'un médicament qui améliore la perfusion rétinienne pour préparer une composition pharmaceutique pour améliorer les défauts du champ visuel associés à l'administration concomitante ou ultérieure d'un médicament GABAergique à un mammifère qui en a besoin.

2. Utilisation selon la revendication 1, dans laquelle le médicament GABAergique est du gamma-vinyl GABA.

3. Utilisation selon la revendication 1, dans laquelle le médicament qui améliore la perfusion rétinienne est un bêtabloquant.

4. Utilisation selon la revendication 1, dans laquelle le médicament qui améliore la perfusion rétinienne est un analogue de la prostaglandine.

5. Utilisation selon la revendication 1, dans laquelle le médicament qui améliore la perfusion rétinienne est un agoniste adrénergique.

6. Utilisation selon la revendication 1, dans laquelle le médicament qui améliore la perfusion rétinienne est un agoniste cholinergique.

7. Utilisation selon la revendication 1, dans laquelle le médicament qui améliore la perfusion rétinienne est un inhibiteur de l'anhydrase carbonique.

8. Utilisation selon la revendication 1, dans laquelle le médicament qui améliore la perfusion rétinienne est un agoniste alpha-adrénergique.

9. Utilisation selon la revendication 1, dans laquelle le médicament qui améliore la perfusion rétinienne est choisi dans le groupe constitué de la pentoxifylline, des dérivés de la puérarine, des dérivés du nitropyrazole, des inhibiteurs de l'oxyde nitrique synthase et des combinaisons de ceux-ci.

10. Utilisation selon la revendication 1, dans laquelle le mammifère est un être humain.

11. Composition comprenant un médicament GABAergique et une quantité efficace d'un médicament qui améliore la perfusion rétinienne.

12. Composition selon la revendication 11, dans laquelle le médicament qui améliore la perfusion rétinienne est un bêtabloquant.

13. Composition selon la revendication 11, dans laquelle le médicament qui améliore la perfusion rétinienne est un analogue de la prostaglandine.

14. Composition selon la revendication 11, dans laquelle le médicament qui améliore la perfusion rétinienne est un agoniste adrénergique.

15. Composition selon la revendication 11, dans laquelle le médicament qui améliore la perfusion rétinienne est un agoniste cholinergique.

16. Composition selon la revendication 11, dans laquelle le médicament qui améliore la perfusion rétinienne est un inhibiteur de l'anhydrase carbonique.

17. Composition selon la revendication 11, dans laquelle le médicament qui améliore la perfusion rétinienne est un agoniste alpha-adrénergique.

18. Composition selon la revendication 11, dans laquelle le médicament qui améliore la perfusion rétinienne est choisi dans le groupe constitué de la pentoxifylline, des dérivés de la puérarine, des dérivés du nitropyrazole, des inhibiteurs de l'oxyde nitrique synthase et des combinaisons de ceux-ci.

19. Composition selon la revendication 11, dans laquelle le médicament GABAergique est du gamma-vinyl GABA.
